# DEMANDE DE BREVET EUROPEEN

(11) **EP 3 868 361 A1**
(43) Date de publication de la demande: **25.08.2021**
(21) Numéro de dépôt: 20158625.2
(22) Date de dépôt: 20.02.2020
(51) Int. Cl.: A61K 9/00, A61K 33/00, A61K 45/06

(54) **MÉLANGE GAZEUX INHALABLE POUR TRAITER LES DOULEURS CHRONIQUES CHEZ DES PATIENTS SOUS OPIOÏDES**

(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: LECOURT, Laurent, 94250 Gentilly (FR); BESSIERE, Patrick, 78354 Les Loges en Josas (FR); HOUETO, Patrick, 78354 Les Loges en Josas (FR); RAMIREZ, Juan-Fernando, 78354 Les Loges en Josas (FR); DELVAL, Cécile, 78354 Les Loges en Josas (FR)
(74) Mandataire: Air Liquide

(57) **Abrégé**

L'invention concerne un mélange gazeux contenant 50% de protoxyde d'azote (N₂O) et au moins 20% d'oxygène (O₂) (% molaires) pour une utilisation en tant que médicament inhalable pour traiter des douleurs chroniques chez un patient humain sous opioïde(s), en particulier un patient réfractaire aux opioïdes.

## Description

L'invention concerne un mélange gazeux contenant 50% de protoxyde d'azote (% mol.) et de l'oxygène, de préférence 50% d'oxygène (% mol.), utilisé en tant que médicament inhalable pour traiter des patients sous opioïde(s), en particulier des patients réfractaires aux opioïdes, c'est-à-dire des patients souffrant de douleurs chroniques, en particulier des douleurs chroniques neuropathiques périphériques, et traités par ailleurs par administration d'un ou plusieurs opioïdes, pendant une durée longue allant jusqu'à plusieurs mois, mais continuant à avoir des douleurs chroniques malgré leur traitement.

Les douleurs chroniques peuvent avoir un retentissement important sur la qualité de vie de nombreux patients, en particulier leur activité fonctionnelle, leur sommeil, leurs niveaux d'anxiété et de dépression... Les patients souffrant de telles douleurs chroniques, en particulier de neuropathies périphériques, doivent être pris en charge thérapeutiquement, c'est-à-dire suivre des traitements médicamenteux de fond visant à diminuer ou à éliminer leurs douleurs.

A cette fin, il est recommandé de leur administrer d'abord des traitements médicamenteux de première intention, à savoir des antidépresseurs tricycliques, des antidépresseurs inhibiteurs de la recapture de la sérotonine et de la noradrénaline (e.g. duloxétine) ou encore des antiépileptiques agissant sur les canaux sodiques ou calciques (e.g. gabapentine, prégabaline). Toutefois, il a été constaté que ces médicaments ne sont pas efficaces chez certains patients, chez qui les douleurs persistent ou chez qui les effets secondaires de ces traitements sont difficilement supportables, i.e. mauvaise tolérance.

Il est alors nécessaire de leur administrer d'autres médicaments, en particulier des opioïdes qui sont recommandés après échec des traitements de première intention, que ce soit en monothérapie ou en association.

Cependant, les opioïdes présentent des inconvénients pour les patients.

Tout d'abord, ils présentent une certaine toxicité pour les patients, en particulier quand ils sont utilisés en chronique, à savoir des risques de tolérance et de dépendance pouvant conduire à une toxicomanie, voire parfois à une mort par overdose.

Ensuite, malgré ces traitements par opioïdes à dose optimale, on a constaté que la majorité des patients traités (>50%) n'a pas un soulagement suffisant de ces douleurs chroniques, c'est-à-dire que les patients continuent à souffrir de douleurs chroniques.

Par ailleurs, on connait FR-A-2981276 décrivant l'utilisation de xénon ou de N₂O pour traiter des douleurs neuropathiques particulières, à savoir celles induites par des sels de platine (i.e., cisplatine, oxaliplatine, carboplatine) ou une autre substance anticancéreuse chez une population particulière de patients, à savoir des patients atteints d'un cancer. Des teneurs allant jusqu'à 70% en volume de xénon ou de N₂O sont données mais il est précisé que les teneurs préférentielles sont de moins de 30% en volume.

En outre, FR-A-2944969 enseigne l'usage de N₂O pour traiter des douleurs neuropathiques chez un patient, en une teneur comprise entre 5 et 45% en volume, de préférence moins de 35% en volume.

Aucun de ces documents antérieurs ne concerne la population particulière susmentionnée, à savoir les patients difficiles à traiter et qui requièrent une administration d'opioïdes au long cours, c'est-à-dire de patients souffrant de douleurs chroniques, en particulier neuropathiques périphériques, et déjà traités par administration répétée d'un ou plusieurs opioïdes.

On connait par ailleurs EP-A-2131812 qui décrit l'usage de N₂O et d'oxygène et/ou d'autres composés pour lutter de manière préventive contre les hyperalgésies post-opératoires, notamment induites par des opioïdes. Les teneurs en N₂O préconisées sont de 5 à 15% en volume. Toutefois, le mélange gazeux est à utiliser dans le cadre d'une procédure d'anesthésie. Le mécanisme d'action les populations et les lieux d'utilisations sont très différents de la présente indication. On comprend aisément que ce type de mélange gazeux n'est donc pas adapté au traitement de patients hors anesthésie, par exemple à leur domicile ou soigné en ambulatoire dans un bâtiment hospitalier, ni ne sont efficaces sur le long terme, typiquement sur plusieurs semaines.

Un problème est donc de pouvoir disposer d'un médicament amélioré permettant de soulager efficacement les patients sous opioïde(s), en particulier les patients réfractaires aux opioïde(s), souffrant de douleurs chroniques, en particulier de douleurs chroniques neuropathiques périphériques.

En d'autres termes, il existe un besoin médical persistant dans la prise en charge des douleurs chroniques, en particulier de douleurs chroniques neuropathiques périphériques, notamment par des traitements plus efficaces et mieux tolérés par les patients que les stratégies actuelles, en particulier les traitements basés sur une administration répétée d'opioïde(s) et ce, notamment en vue d'améliorer le rapport bénéfice/risque de ces traitements.

Dit encore autrement, il est souhaitable de pouvoir améliorer l'efficacité et la tolérance de patients souffrants de douleur chroniques, en particulier de douleurs chroniques neuropathiques périphériques, lesquels patients sont sous opioïdes, en particulier les patients réfractaires au traitement par opioïdes, c'est-à-dire traités par administration préalable d'au moins un traitement à base d'un ou plusieurs opioïdes mais chez lesquels le ou les opioïdes ne sont pas ou peu efficaces et/ou mal tolérés.

Une solution de l'invention concerne un mélange gazeux contenant 50% de protoxyde d'azote (N₂O) et au moins 20% d'oxygène (O₂) (% mol.) pour une utilisation en tant que médicament inhalable pour traiter des douleurs chroniques chez un patient humain sous opioïde (i.e. un ou plusieurs opioïdes), de préférence un patient réfractaire aux opioïde(s).

Dans le cadre de la présente invention :
- on utilise la dénomination « patient sous opioïde(s) » pour désigner une catégorie particulière de patients, à savoir ceux traités un ou des opioïdes ; toutefois, ce traitement par opioïde(s) ne fait pas partie de la présente invention mais sert uniquement à identifier la population cible.
- on appelle « patient réfractaire aux opioïdes », une personne chez laquelle un traitement à base d'un ou plusieurs opioïdes, qui lui a été administré préalablement pendant une durée longue, typiquement plusieurs jours, semaines ou mois, pour tenter de soulager ses douleurs chroniques, n'est pas parvenu à produire un effet suffisant pour diminuer lesdites douleurs chroniques, donc chez lequel le ou les opioïdes ne sont pas ou peu efficaces et/ou mal tolérés, notamment du fait d'effets secondaires possibles liés à une posologie incompatible avec le patient considéré.
- le ou les opioïdes ont été administrés en traitement de fond de la douleur en particulier pendant des durées longues, typiquement pendant plusieurs jours, semaines ou mois, avec une ou des administrations journalières de ces composés médicamenteux ; toutefois, ce traitement par opioïde(s) ne fait pas partie de la présente invention.
- les proportions de composés (N₂O, O₂) dans le mélange gazeux sont exprimées en pourcentages molaires (i.e., % mol.).

Selon le mode de réalisation considéré, le mélange gazeux utilisé en tant que médicament inhalable selon l'invention peut comprendre l'une ou plusieurs des caractéristiques suivantes :
- il contient au moins 30% d'oxygène, de préférence au moins 40% d'oxygène.
- il contient au maximum 50% d'oxygène environ.
- il contient de l'oxygène, du N₂O et de l'azote (N₂).
- il est constitué de 50% de protoxyde d'azote (N₂O) et 50% d'oxygène (O₂). Ce mélange équimolaire (50%/50%) est aussi appelé MEOPA)
- bien entendu, même si cela n'est pas souhaitable et/ou désiré, le mélange gazeux, tel le MEOPA, peut contenir éventuellement des impuretés inévitables résultant du procédé de fabrication du mélange, par exemple des impuretés de type CO, CO₂, H₂O, NOx... Leur teneur éventuelle est maintenue aussi faible que possible et vérifiée pour garantir une innocuité du mélange gazeux N₂O/O₂ et une conformité à son statut de médicament.
- le protoxyde d'azote est le principe actif du mélange gazeux.
- le patient est traité uniquement par un ou des opioïdes.
- le ou les opioïdes sont choisis parmi les composés opioïdes suivants : tramadol, codéine, oxycodone, tapentadole, fentanyl, remifentanyl, morphine ou tout autre dérivé naturel ou chimique de la morphine.
- le patient est réfractaire à un traitement par plusieurs opioïdes.
- le patient est réfractaire à un traitement par au moins du tramadol.
- les douleurs chroniques sont des douleurs neuropathiques.
- les douleurs chroniques sont des douleurs présentant au moins une composante périphérique, telle une douleur mixte par exemple.
- les douleurs chroniques sont des douleurs neuropathiques périphériques.
- le patient humain est douloureux chronique.
- les douleurs chroniques sont des douleurs neuropathiques périphériques liées à et/ou résultant d'une (ou des) lésion ou irritation d'un ou plusieurs nerfs périphériques.
- la lésion ou irritation du ou des nerfs périphériques est d'origine traumatique, toxique, métabolique, ischémique, immuno-allergique ou infectieuse.
- les douleurs neuropathiques chroniques périphériques résultent ou sont liées à une lésion ou irritation d'un ou plusieurs nerfs périphériques.
- les douleurs neuropathiques chroniques périphériques sont choisies notamment parmi les douleurs post-zostériennes, les douleurs des neuropathies diabétiques, les polyneuropathies, et les douleurs post-traumatiques ou post-opératoires.
- les douleurs sont spontanées et/ou provoquées.
- les douleurs spontanées sont continues et/ou superficielles (e.g. sensation de brûlures) ou profondes (e.g. sensation d'un serrage par un étau ou de compression), avec ou sans survenue d'accès paroxystiques (i.e. sensation de décharge électrique) et de paresthésies/dysesthésies.
- les douleurs provoquées se caractérisent par une hypersensibilité au chaud, au froid ou au toucher.
- le mélange gazeux n'est pas administré pendant une anesthésie, c'est-à-dire qu'il est destiné à des patients conscients, i.e. non-anesthésiés. Dit autrement, le mélange gazeux selon l'invention n'est pas destiné à être utilisé dans le cadre d'une procédure d'anesthésie chez des patients devant subir une telle procédure d'anesthésie.
- le mélange gazeux est prêt à l'emploi et conditionné dans un récipient de gaz, en particulier une bouteille de gaz.
- alternativement, le mélange gazeux est réalisé sur-site au moyen d'un mélangeur de gaz alimenté en O₂ et N₂O.

L'invention concerne aussi une utilisation d'un mélange gazeux N₂O/O₂ contenant 50% de protoxyde d'azote (N₂O) et au moins 20% d'oxygène (O₂) (% mol.) selon l'invention, de préférence un mélange équimolaire 50%/50% N₂O/O₂, pour fabriquer un médicament inhalable permettant de soulager efficacement les patients sous opioïdes (i.e. un ou plusieurs opioïdes) souffrant de douleurs chroniques, en particulier de douleurs neuropathiques chroniques périphériques (DNCP), de préférence des patients réfractaires à un traitement par opioïdes, c'est-à-dire des personnes non-réceptives à un traitement de fond à base d'un ou plusieurs opioïdes.

Par ailleurs, l'invention porte aussi une méthode de traitement permettant de soulager un patient souffrant de douleurs chroniques, en particulier de douleurs neuropathiques chroniques périphériques (DNCP), ledit patient étant en outre sous un ou plusieurs opioïdes, en particulier un patient réfractaire à un traitement par opioïde(s), dans laquelle on administre par inhalation audit patient un mélange gazeux N₂O/O₂ contenant 50% de protoxyde d'azote (N₂O) et au moins 20% d'oxygène (O₂) (% mol.) selon l'invention, de préférence un mélange équimolaire 50%/50% N₂O/O₂.

Selon le mode de réalisation considéré, la méthode de traitement de l'invention peut comprendre l'une ou plusieurs des caractéristiques suivantes :
- le mélange gazeux est administré au moins une fois par jour.
- le mélange gazeux est administré au moins une fois par jour pendant 1 à plusieurs jours, de préférence plusieurs jours consécutifs, par exemple de 1 à 3 jours consécutifs.
- on répète l'administration à des intervalles de temps d'au moins 1 semaine, de préférence d'au moins 2 à 4 semaines, en fonction de la réponse au traitement.
- la séquence d'administration est répétée pendant plusieurs mois, voire plusieurs années.
- la séquence d'administration se poursuit jusqu'à disparition ou atténuation forte des effets du mélange gazeux, c'est-à-dire tant que le traitement est bien toléré par le patient et/ou de que son efficacité est suffisante.
- le mélange gazeux est administré pendant au moins 30 min, de préférence au moins 45 min, avantageusement au moins 1 heure environ à chaque administration, typiquement entre 55 min et 65 min, c'est-à-dire pendant une durée suffisante pour obtenir un effet long-terme sur plusieurs semaines.
- le mélange gazeux est administré à un débit compris entre 1 et 25 L/min, de préférence entre 2 et 16 L/min.
- le patient est un adulte ou un enfant (i.e. > 2 ans).

Selon un autre aspect, l'invention porte aussi sur un récipient de gaz sous pression contenant un mélange gazeux selon l'invention, en particulier un mélange équimolaire 50%/50% N₂O/O₂.

Selon encore un autre aspect, l'invention porte aussi sur un système d'administration d'un mélange gazeux N₂O/O₂ selon l'invention comprenant un récipient de gaz contenant le mélange gazeux N₂O/O₂ à administrer, et une interface respiratoire de fourniture de gaz au patient.

Selon le mode de réalisation considéré, le système d'administration selon l'invention peut comprendre l'une ou plusieurs des caractéristiques suivantes :
- le récipient de gaz est équipé d'un bloc robinet de distribution de gaz, de préférence un bloc robinet à détendeur intégré ou RDI.
- le récipient de gaz a un corps cylindrique, de préférence en métal ou en matériaux composites, notamment en alliage d'aluminium ou en acier.
- le récipient de gaz est équipé d'un bloc robinet de distribution de gaz protégé par un capotage de protection, aussi appelé « chapeau », en polymère ou en métal.
- l'interface respiratoire de fourniture de gaz au patient est un masque nasal, facial (i.e. bucco-nasal) ou narinaire, ou un embout buccal, ou toute autre interface de distribution de gaz adaptée à la fournir de gaz aux voies aériennes d'un individu.
- l'interface respiratoire est reliée fluidiquement au récipient de gaz par un tuyau flexible ou analogue servant à véhiculer le gaz.
- le récipient de gaz contient un mélange gazeux N₂O/O₂ comprenant 50% de protoxyde d'azote (N₂O) et au moins 20% d'oxygène (O₂) (% mol.), de préférence au moins 30% d'oxygène (O₂), typiquement un mélange équimolaire 50%/50% N₂O/O₂.

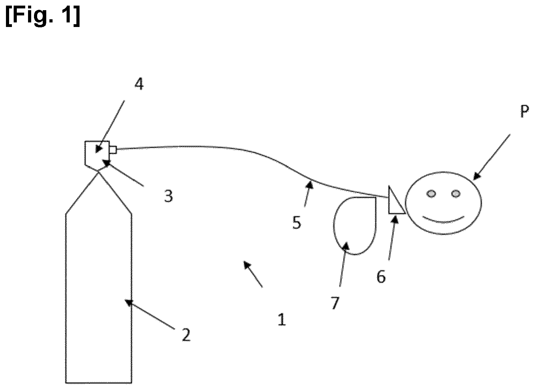

L'invention va maintenant être mieux comprise grâce à la description détaillée suivante, faite à titre illustratif mais non limitatif, à la Figure 1 annexée et aux exemples comparatifs consignés ci-après, où la Figure 1 schématise un système d'administration d'un mélange gazeux selon l'invention.

La Figure 1 schématise un mode de réalisation d'un système d'administration 1 d'un mélange gazeux N₂O/O₂ selon l'invention à un patient humain sous opioïde(s), c'est-à-dire traité par ailleurs par administration d'un ou plusieurs opioïdes, lequel patient souffre de douleurs chroniques, en particulier de douleurs chroniques neuropathiques, en particulier un patient réfractaire aux opioïdes.

De préférence, le mélange gazeux est constitué de 50% de protoxyde d'azote (N₂O) et 50% d'oxygène (O₂), et éventuellement des impuretés inévitables résultant par exemple du procédé de fabrication du mélange gazeux. Ce mélange gazeux binaire équimolaire N₂O/O₂ (50%/50%) est appelé MEOPA pour Mélange Equimolaire Oxygène Protoxyde d'Azote.

Ce système d'administration 1 comprend une bouteille de gaz 2 équipée d'un robinet 3 à détendeur intégré (RDI) contenant le mélange gazeux N₂O/O₂, tel le MEOPA, qui y est conditionné à une pression pouvant atteindre 300 bar abs, appelée « pression haute ». Le RDI 3 permet de réduire la pression du mélange gazeux N₂O/O₂ jusqu'à une pression plus basse, appelée « pression basse » ou « pression de sortie », par exemple de 5 bar abs ou moins, qui est délivré par un raccord 4 de sortie du RDI 3.

Le mélange N₂O/O₂ est acheminé jusqu'au patient P, via un tuyau flexible 5, qui est en communication fluidique, d'une part, avec le raccord de sortie 4 du RDI 3 et, d'autre part, avec une interface 6 de distribution du gaz, tel un masque respiratoire.

Préférentiellement, un réservoir-tampon 7, tel un ballon-flexible, peut être inséré en amont de l'interface respiratoire 6 pour faciliter l'administration du gaz au patient P.

Bien entendu, on peut aussi utiliser un autre dispositif d'administration dès lors qu'il est adapté à la fourniture d'un mélange N₂O/O₂ au patient P à soigner.

Le mélange gazeux N₂O/O₂ est un médicament inhalable servant à traiter les douleurs chroniques, en particulier neuropathiques, préférentiellement d'origine périphérique, chez le patient P qui est par ailleurs traités avec un ou des opioïdes, lesquels opioïdes ne permettant pas, à eux seuls, de faire disparaître les douleurs du patient ou sont mal tolérés par celui-ci, en particulier un patient réfractaire aux opioïdes.

Le ou les opioïdes sont par exemple le tramadol, la codéine, l'oxycodone, le tapentadol, le fentanyl, le rémifentanil et/ou la morphine et ses dérivés.

D'une façon générale, les douleurs chroniques neuropathiques se distinguent par leur physiopathologie et leur expression clinique. Elles ont été définies par l'International *Association for the Study of Pain,* comme des douleurs secondaires à une lésion ou à une maladie affectant le système somato-sensoriel. Elles peuvent être d'origine périphérique ou d'origine centrale.

Dans le cadre de la présente invention, on considère préférentiellement les douleurs chroniques neuropathiques ayant une composante périphérique, aussi appelées douleurs neuropathiques chroniques périphériques (DNCP). Celles-ci surviennent par exemple à la suite d'une lésion ou irritation d'origine traumatique, toxique, métabolique, ischémique, immuno-allergique ou infectieuse des nerfs périphériques. Elles sont choisies notamment parmi les douleurs post-zostériennes, les douleurs des neuropathies diabétiques, les polyneuropathies, et les douleurs post-traumatiques ou post-opératoires.

Elles se caractérisent par des douleurs spontanées et/ou provoquées.

Les douleurs spontanées peuvent être continues et/ou superficielles (e.g. sensation de brûlures) ou profondes (e.g. sensation d'un serrage par un étau ou de compression) et peuvent engendrer une survenue d'accès paroxystiques (i.e. sensation de décharge électrique) et de paresthésies/dysesthésies.

Les douleurs provoquées se caractérisent quant à elles par des hypersensibilité au chaud au froid, au toucher et sont aussi appelées « hyperalgies » ou « allodynies ».

Afin de montrer l'efficacité du médicament inhalable N₂O/O₂ selon l'invention, les exemples comparatifs suivants ont été réalisés.

### Exemples

Des essais comparatifs ont été réalisés dans 2 pays et dans 22 hôpitaux, en utilisant du MEOPA (50/50% N₂O/O₂) selon l'invention et de l'air médical servant de témoin (i.e. gaz placebo). Ces mélanges gazeux ont été administrés par inhalation à des patients adultes souffrant de douleurs neuropathiques chroniques périphériques (DNCP) et traités ou non par opioïdes en traitement de fond, en utilisant un même système d'administration tel que décrit en [Fig. 1].

Les patients ont été tirés au sort pour recevoir soit le MEOPA soit de l'air médical. Toutefois, les patients ne sont pas informés du gaz qu'ils inhalent. Les patients reçoivent les gaz testés alors qu'ils sont éveillés et conscients (i.e. hors toute anesthésie).

Les traitements ont été administrés pendant 1 heure par jour pendant 3 jours consécutifs. Chaque patient de l'étude était suivi pendant 4 semaines et devait compléter tout au long du suivi plusieurs questionnaires (i.e. NRS, SF-12, NPSI, PGIC) permettant d'évaluer l'évolution :
- de l'intensité de la douleur. Le questionnaire NRS permet aux patients d'évaluer l'intensité de leur douleur sur une échelle allant de 0 (absence de douleur) à 10 (douleur la plus forte possible).
- de la qualité de vie. Le questionnaire SF-12 représente une échelle de qualité vie. Elle comporte 8 dimensions permettant d'évaluer sur les 4 semaines précédant l'administration, la composante physique et la composante mentale de la qualité de vie de chaque patient.
- des différents composantes de la douleur neuropathique. Le questionnaire NPSI permettant de caractériser les différentes composantes de la douleur. Il comprend 12 questions donnant un score total et 5 sous-scores représentant les dimensions suivantes: douleurs spontanées superficielles à type de brûlures, douleurs spontanées profondes à type de compression, d'étau, douleurs paroxysmales à type de décharges électriques, de coups de couteau, paresthésies et dysesthésies à type de fourmillements, douleurs provoquées par les frottements, et contact du froid.
- du ressenti douloureux global. Le questionnaire PGIC est une échelle qui permet de mesurer l'évolution sous traitement des limitations d'activité, des symptômes, des émotions et de la qualité de vie globale qui sont liés à la condition douloureuse. Cette évolution est mesurée en 7 points allant de "très détériorée" à "très améliorée".

Les résultats des essais sont obtenus sur 221 patients recevant 3 administrations de MEOPA ou de placebo (air médical) dont au moins une administration complète, i.e. durée d'administration allant de 55 à 65 minutes avec des pauses cumulées n'excédant pas 5 minutes, et ayant fait au moins 4 évaluations de l'intensité de leur douleur durant la première semaine après le traitement.

Les résultats sont consignés dans les Tableaux ci-après où :
[Tableau 1] donne les résultats de l'évolution de l'intensité de la douleur (score NRS) sur les 4 semaines de suivi sur la population globale des patients DNCP de l'étude,
[Tableau 2] consigne les résultats de l'évolution de l'intensité de la douleur (score NRS) sur les 4 semaines de suivi chez les patients DNCP de l'étude traités par des opioïdes,
[Tableau 3] liste des opioïdes utilisés chez les patients du [Tableau 2], et
[Tableau 4] donne les résultats de l'évolution de l'intensité de la douleur (score NRS) sur les 4 semaines de suivi chez les patients DNCP de l'étude non traités par opioïde(s).

**[Tableau 1]**

| | MEOPA | AIR (Placebo) | Traitement Diff. (95% Cl) | |
|---|---|---|---|---|
| | (N=103) | (N=118) | | |
| Changements de l'intensité de la douleur (score NRS) après 1 semaine: moyenne (écart-type) | | | | |
| moyenne | - 1.02 (1.5) | -0.71 (1.3) | - 0.22 (-0.59 ; 0.15) | p = 0.25 |
| | | | | |

| Changements après 2 semaines: moyenne (écart-type) | | | | |
|---|---|---|---|---|
| moyenne | - 0.93 (1.6) | - 0.68 (1.4) | - 0.24 (-0.63 ; 0.15) | p = 0.22 |
| | | | | |

| Changements après 3 semaines: moyenne (écart-type) | | | | |
|---|---|---|---|---|
| moyenne | - 0.90 (1.5) | - 0.67 (1.4) | - 0.23 (-0.63 ; 0.17) | p = 0.26 |
| | | | | |

| Changements après 4 semaines: moyenne (écart-type) | | | | |
|---|---|---|---|---|
| moyenne | - 0.83 (1.4) | - 0.67 (1.4) | - 0.17 (-0.56 ; 0.22) | p = 0.39 |

Les résultats contenus dans [Tableau 1] concernent la population globale de l'étude et montrent l'absence de différence statistiquement significative concernant la réduction de l'intensité de la douleur de la semaine 1 à la semaine 4, entre les patients traités par MEOPA et les patients traités par de l'air médical.

**[Tableau 2]**

| | ***Groupe opioides*** | ***Groupe opioides*** | Traitement Diff. (95% Cl) | |
|---|---|---|---|---|
| | MEOPA | AIR (Placebo) | | |
| | (N=43) | (N=49) | | |
| Changements de l'intensité de la douleur (score NRS) après 1 semaine: moyenne (écart-type) | | | | |
| moyenne | - 1.22 (1.5) | - 0.45 (1.0) | - 0.77 (-1.29 ; - 0.26) | **p = 0.004** |
| Changements après 2 semaines: moyenne (écart-type) | | | | |
| moyenne | - 1.19 (1.5) | - 0.35 (0.9) | - 0.84 (-1.36 ; - 0.33) | **p = 0.002** |
| Changements après 3 semaines: moyenne (écart-type) | | | | |
| moyenne | - 1.08 (1.5) | - 0.20 (1.0) | - 0.86 (-1.39 ; - 0.32) | **p = 0.002** |
| Changements après 4 semaines: moyenne (écart-type) | | | | |
| moyenne | - 0.99 (1.3) | - 0.30 (1.0) | - 0.66 (-1.16 ; - 0.17) | **p = 0.009** |

Les résultats contenus dans [Tableau 2] concernent la population des patients de l'étude traités de façon chronique par opioïdes et montrent, en faveur du MEOPA, des différences statistiquement significatives concernant la réduction de l'intensité de la douleur de la semaine 1 à la semaine 4, entre les patients traités par MEOPA et les patients traités par de l'air médical (i.e. placébo).

**[Tableau 3]**

| Opioides | Total | % |
|---|---|---|
| Tramadol | 29 | 31,5 |
| Papaver somniferum (+ paracétamol) | 13 | 14,1 |
| Codéine (+ paracétamol) | 12 | 13 |
| Tramadol (+ paracétamol) | 11 | 12 |
| Oxycodone | 9 | 9,8 |
| Morphine | 7 | 7,6 |
| Caféine + Papaver somniferum (+ paracétamol) | 6 | 6,5 |
| Fentanyl | 6 | 6,5 |
| Naloxone + Tilidine | 3 | 3,3 |
| Dihydrocodéine | 2 | 2,2 |
| Naloxone + Oxycodone | 1 | 1,1 |

**[Tableau 4]**

| | MEOPA (N=60) | AIR (Placebo) (N=69) | Traitement Diff. (95% | Cl) |
|---|---|---|---|---|
| Changements de l'intensité de la douleur (score NRS) après 1 semaine: moyenne (écart-type) | | | | |
| moyenne | - 0.88 (1.5) | -1.1 (1.4) | + 0.20 (- 0.3 ; 0.72) | **p = 0.44** |
| Changements après 2 semaines: moyenne (écart-type) | | | | |
| moyenne | - 0.73 (1.6) | - 0.93 (1.6) | + 0.22 (- 0.34 ; 0.78) | **p = 0.44** |
| Changements après 3 semaines: moyenne (écart-type) | | | | |
| moyenne | - 0.8 (1.5) | - 1.0 (1.6) | + 0.25 (- 0.34 ; 0.82) | **p = 0.38** |
| Changements après 4 semaines: moyenne (écart-type) | | | | |
| moyenne | - 0.7 (1.5) | - 0.90 (1.7) | + 0.22 (- 0.35 ; 0.78) | **p = 0.45** |

Les résultats contenus dans [Tableau 4] concernent la population des patients de l'étude non traités par opioïdes. Ils montrent clairement l'absence de différence statistiquement significative concernant la réduction de l'intensité de la douleur de la semaine 1 à la semaine 4, entre les patients traités par MEOPA et les patients traités par de l'air médical (i.e. placebo).

On constate, au vu de ces Tableaux, que, de façon surprenante, une efficacité supérieure du MEOPA comparativement à l'air médical (placebo) sur de multiples critères associés à l'évaluation de la douleur chronique et des symptômes en relation avec cette symptomatologie douloureuse (critères NRS, NPSI, PGIC, SF12) chez les patients traités de façon chronique par opioïdes (cf. [Tableau 3]), alors que sur la population globale (cf. [Tableau 1]) et sur la population des patients non traités par opioïdes (cf. [Tableau 4]), ces mêmes critères montrent peu ou pas de significativité.

En d'autres termes, l'usage du médicament selon l'invention contenant 50% de N₂O montre une efficacité notable uniquement chez les patients souffrant de DNCP et traités par opioïdes mais pas chez ceux qui ne sont pas sous opioïdes.

Une telle constatation est très surprenante car elle signifie que le médicament selon l'invention contenant 50% de N₂O a une efficacité ciblée dans la lutte contre la douleur sur une (sous-)population de patients particulière (i.e. patients traités par opioide(s)) mais pas sur la population générale de patients souffrant de DNCP.

Il est de plus constaté que, dans cette population particulière de patients traités par opioide(s), une persistance de l'efficacité du médicament inhalable selon l'invention, ici du MEOPA, pendant une durée de plusieurs semaines, à savoir au moins 4 semaines, sans ré-administration du MEOPA (cf. [Tableau 3]). Ce deuxième effet surprenant n'existe avec aucun des autres traitements de fond reçus par les patients, à savoir traitements locaux, par voie générale, ou autres.

Il est aussi à souligner que l'efficacité bien plus marquée du MEOPA sur la sous-population de patients sous opioïde(s) ne se fait pas au détriment de la tolérance du MEOPA puisque la tolérance du MEOPA restent satisfaisante sur ces patients.

De façon générale, un mélange gazeux contenant 50% de N₂O selon l'invention permet de satisfaire un besoin médical persistant dans la prise en charge des douleurs chroniques, en particulier neuropathiques périphériques, chez les patients sous opioïde(s), en particulier des patients réfractaires aux opioïdes, en conduisant à un traitement plus efficace et mieux toléré par les patients que les stratégies classiques, en particulier les traitements basés sur une administration répétée d'opioïde(s), ce qui permet en outre d'améliorer le rapport bénéfice/risque pour les patients, en particulier les patients réfractaires aux opioïdes.

## Revendications

1. Mélange gazeux contenant 50% de protoxyde d'azote (N₂O) et au moins 20% d'oxygène (O₂) (% molaires) pour une utilisation en tant que médicament inhalable pour traiter des douleurs chroniques chez un patient humain sous opioïde(s).

2. Mélange gazeux selon la revendication 1, **caractérisé en ce que** le patient est réfractaire aux opioïdes.

3. Mélange gazeux selon la revendication 1, **caractérisé en ce qu'**il contient au moins 30% d'oxygène, de préférence au moins 40% d'oxygène (% molaires).

4. Mélange gazeux selon l'une des revendications 1 ou 3, **caractérisé en ce qu'**il contient de l'oxygène, du N₂O et de l'azote (N₂).

5. Mélange gazeux selon l'une des revendications 1 ou 3, **caractérisé en ce qu'**il est constitué de 50% de protoxyde d'azote (N₂O) et 50% d'oxygène (O₂) (% molaires).

6. Mélange gazeux selon l'une des revendications 1 ou 2, **caractérisé en ce que** l'opioïde est choisi parmi tramadol, codéine, oxycodone, tapentadol, fentanyl, remifentanyl et morphine.

7. Mélange gazeux selon la revendication 1, **caractérisé en ce que** les douleurs chroniques sont des douleurs neuropathiques périphériques.

8. Mélange gazeux selon l'une la revendication 7, **caractérisé en ce que** les douleurs neuropathiques chroniques périphériques résultent ou sont liées à une lésion ou irritation d'un ou plusieurs nerfs périphériques.

9. Mélange gazeux selon la revendication 1, **caractérisé en ce que** le patient est un adulte ou un enfant.

10. Mélange gazeux selon l'une des revendications 1 à 9, **caractérisé en ce qu'**il est formulé pour une administration d'au moins une fois par mois, de préférence 1 à 3 fois par mois.

11. Mélange gazeux selon l'une des revendications 1 à 10, **caractérisé en ce qu'**il est formulé pour une administration d'au moins une fois par jour pendant une durée d'administration d'au moins 30 min, de préférence de 55 min à 65 min.

12. Mélange gazeux selon l'une des revendications 1 à 11, **caractérisé en ce qu'**il est formulé pour une administration répétée pendant plusieurs mois, de préférence plusieurs années.

13. Mélange gazeux selon l'une des revendications 1 à 12, **caractérisé en ce qu'**il formulé pour une administration à un débit compris entre 1 et 25 L/min.

14. Récipient de gaz sous pression contenant un mélange gazeux selon la revendication 1.

15. Système d'administration d'un mélange gazeux N₂O/O₂ selon l'une des revendications précédentes, comprenant un récipient de gaz selon la revendication 14 contenant le mélange gazeux N₂O/O₂ à administrer, et une interface respiratoire de fourniture de gaz au patient.
